(19) Europäisches Patentamt / European Patent Office / Office européen des brevets

(11) **EP 3 863 728 B1**

(12) **EUROPEAN PATENT SPECIFICATION**

(45) Date of publication and mention
of the grant of the patent:
**15.07.2026 Bulletin 2026/29**

(21) Application number: **19784151.3**

(22) Date of filing: **10.10.2019**

(51) International Patent Classification (IPC):
*A61Q 19/08* (2006.01)   *A61K 47/36* (2006.01)
*A61K 47/32* (2006.01)   *A61K 47/14* (2017.01)
*A61K 9/06* (2006.01)   *A61K 9/00* (2006.01)
*A61K 8/81* (2006.01)   *A61K 8/73* (2006.01)
*A61K 8/37* (2006.01)   *A61K 8/04* (2006.01)
*A61K 38/18* (2006.01)

(52) Cooperative Patent Classification (CPC):
**A61Q 19/08; A61K 8/042; A61K 8/37; A61K 8/735;
A61K 8/8129; A61K 9/0014; A61K 9/06;
A61K 38/185; A61K 47/14; A61K 47/32;
A61K 47/36**

(86) International application number:
**PCT/IB2019/058641**

(87) International publication number:
**WO 2020/075108 (16.04.2020 Gazette 2020/16)**

(54) **PEPTIDES FOR USE IN TREATING AND PREVENTING SKIN AGING AND PHOTO-AGING**

PEPTIDE ZUR VERWENDUNG ZUR BEHANDLUNG UND VERHINDERUNG VON
HAUTALTERUNG UND ALTERUNG DURCH LICHTEINFLUSS

PEPTIDES POUR L'UTILISATION POUR TRAITER ET PREVENIR LE VIEILLISSEMENT DE LA
PEAU ET LE PHOTO-VIEILLISSEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.10.2018 IT 201800009384**

(43) Date of publication of application:
**18.08.2021 Bulletin 2021/33**

(73) Proprietor: **Dompé Farmaceutici S.p.A.
20122 Milano (IT)**

(72) Inventor: **PELLEGRINI, Pablo
20123 Milano (IT)**

(74) Representative: **PGA S.p.A.
Via Mascheroni, 31
20145 Milano (IT)**

(56) References cited:
**WO-A1-98/49308       WO-A2-2013/065078
WO-A2-99/39728       CN-A- 107 286 233
CN-A- 107 973 848     KR-A- 20100 058 858
KR-A- 20170 049 299**

Remarks:
The complete document including Reference
Table(s) and the Sequence Listing(s) can be
downloaded from the EPO website

**Description**

**FIELD OF THE INVENTION**

The present invention relates to a peptide for cosmetic application. In particular, the present invention relates to the peptide having Seq. ID No. 1 able to reduce the skin aging and photo-aging. More in particular, the present invention also relates to a cosmetic composition comprising the peptide having Seq. ID No. 1 and at least one cosmetically acceptable excipient.

**BACKGROUND OF THE INVENTION**

Nerve growth factor (NGF) is a neurotrophic factor and neuropeptide primarily involved in the regulation of growth, maintenance, proliferation, and survival of certain target neurons.

The NGF was discovered by Prof. Rita Levi-Montalcini, at the Zoology Institute of the Washington University of St. Louis (Levi-Montalcini R., Harvey Lect., 60:217, 1966), and its discovery represented a remarkable advance in the study of the growth and differentiation mechanisms of the nerve cell, as NGF is able to affect the development and preservation of the biological functions of the neurons and their regeneration. For the discovery of this molecule, and for having characterized its biological function both in the peripheral and the central nervous system, in 1986 Prof. R. Levi-Montalcini was awarded the Nobel Prize for Medicine and Physiology. A number of in vitro and in vivo studies have demonstrated the pathophysiological importance of NGF in preventing neuronal damage of surgical, chemical, mechanical and ischemic nature, making it the ideal candidate for use in the treatment of several conditions of the peripheral and central nervous systems (Hefti F., J. Neurobiol., 25: 1418, 1994; Fricker J., Lancet, 349:480, 1997). In fact, since many years ago clinical trials on patients suffering from Parkinson's disease and Alzheimer's disease have been carried out, by intracerebral administration of murine NGF (see, e.g., Olson L. et al., J. Neural Trans.: Parkinson's Disease and Dementia Section, 4: 79, 1992). Results of these studies confirmed the observations made in animal models and showed the absence of possible side effects following administration of murine NGF. This feature was subsequently confirmed for the human recombinant NGF (Petty B.G. et al., Annals of Neurology, 36:244-246, 1994).

These events are mediated by NGF as a result of binding to its two cell-surface receptors, TrkA and p75. Co-expression of the two genes for the p75 receptor and the Trk NGF receptor can potentially lead to greater responsiveness to NGF, and suggests additional levels of regulation for the family of neurotrophin factors (Chao MV, Hempstead BL., "p75 and Trk: a two-receptor system", Trends Neurosci. 1995;18:321-6. Chick embryo sensory neurons display two different classes of neurotrophin receptors, p75 and Trk, with dissociation constants of $10^{-9}$ M and $10^{-11}$ M, respectively, implying that only low occupancy of the higher affinity receptor is required to mediate the biological actions of the neurotrophins (Meakin SO, Shooter EM, "The nerve growth factor family of receptors", Trends Neurosci. 1992;15:323-31.

TrkA is a receptor with tyrosine kinase activity that forms a high-affinity binding site for NGF.

p75 is a transmembrane protein with no identifiable cytoplasmic catalytic domain belonging to the tumor necrosis factor (TNF) receptor superfamily that forms a low-affinity binding site for NGF.

International patent publications WO2004026329A1, WO2011116090, WO2011049758A1 and WO2005019266A2 relate to peptides or antibodies that interact with or bind to human nerve growth factor (NGF) and neutralize the function of NGF, and to the use thereof in the prevention and/or treatment of various diseases and disorders in which NGF activity is detrimental, including sunburn and vitiligo.

Contrary to what suggested by the patent publications cited above, international patent publication WO2013065078A2 disclosed that the topical administration to the skin of preparations containing NGF is effective in achieving an intensification of skin color, that is an increase in pigmentation, in the case of healthy skin, not affected by dyschromatosis, as well as an improvement of dermatological conditions involving skin achromias or hypochromias, as is the case of vitiligo.

The peptide fragment containing the 1-14 sequence of the human NGF (NGF1-14) is known in the art as NGF mimetic, as disclosed in some literature references (P. Di Pietro et al., "Immobilization of Neurotrophin Peptides on Gold Nanoparticles by Direct and Lipid-Mediated Interaction: A New Multipotential Therapeutic Nanoplatform for CNS Disorders", ACS Omega (2017), 2(8), 4071-4079 e C. Satriano et al., "Neurotrophin-mimicking peptides at the biointerface with gold respond to copper ion stimuli", Physical Chemistry Chemical Physics (2016), 18(44), 30595-30604).

On the other hand, the NGF1-14 was demonstrated to only interact with the TrkA receptor, in particular with activation of the PI3/Akt cascade and CREB phosphorylation, without being able to interact with the p75 receptor and to induce ERK1/2 phosphorylation (A. Travaglia et al., "A small linear peptide encompassing the NGF N-terminus partly mimics the biological activities of the entire neurotrophin in PC12 cells", ACS Chem Neurosci. 2015 Aug 19;6(8):1379-92).

Finally, WO99/39728 disclosed that p75 signals for keratinocyte and melanocyte apoptosis when activated alone, but instead signals for cell survival when activated together with receptors of the Trk family. WO 2013/065078, WO 99/39728, KR2017 0049299 disclose nerve growth factor components for the treatment and prevention of disorders which accompany skin ageing. KR 2010 0058858 focuses on a skin anti-ageing cosmetic composition that contains 0.001 -1.0 weight % of glutamylamidoethyl indole as an active ingredient. The glutamylamidoethyl indole promotes synthesis of NFG (nerve growth factor). WO 98/49308, CN 107 286 233, CN 107 973 848 disclose further nerve growth factors for the use for the development, differentiation and regeneration of central and peripheral nerve cells (sequences far exceeding a length of 16 amino acids).

## SUMMARY OF THE INVENTION

Starting from the knowledge derived from the state of the art, sometimes contradictory, the Applicant has investigated the potential use of NGF1-14 for cosmetic application.

NGF1-14 is represented by the following sequence:

Seq. ID No. 1 : SSSHPIFHRGEFSV

During extensive experimentation, the Applicant has surprisingly found that NGF1-14 was able to promote the production of pro-collagen I and elastin, as well as to activate melanocytes for melanin production. This finding suggested the use of NGF1-14 in cosmetic composition for the treatment and prevention of skin aging and photo-aging.

Further, the Applicant has found that NGF1-14 peptide retained all the rh-NGF effects desirable for a dermatological use.

A further advantage found by the Applicant is that NGF1-14, opposite to the whole protein, can be easily synthesized by conventional peptide synthesis approaches and stored under mild conditions and for prolonged times.

These advantages led to a significant reduction of the production costs and made NGF1-14 very appropriate and attractive for a cosmetic use.

The Applicant further found that some modification in the number and type of amino acid of NGF1-14 can be made without substantially altering the activity of NGF1-14.

Accordingly, a first object of the present invention relates to a peptide comprising the sequence ID No. 1, or a sequence having at least 85%, preferably at least 90%, and more preferably at least 95% sequence identity with sequence ID No. 1, and a derivative or salt thereof, for use in the treatment and prevention of skin aging and photo aging, wherein said peptide has a length up to 16 amino acids.

Further, a second object of the present invention relates to a cosmetic composition comprising a peptide comprising the sequence ID No. 1, or a sequence having at least 85%, preferably at least 90%, and more preferably at least 95% sequence identity with sequence ID No. 1, and a derivative or salt thereof, wherein said peptide has a length up to 16 amino acids, and at least one cosmetically acceptable excipient for use in the treatment and prevention of skin aging and photo-aging.

A third object of the present invention relates to a non-therapeutic method for the treatment and prevention of skin aging and photo-aging in a person in need thereof, comprising the topical application of a cosmetic composition comprising a peptide comprising the sequence ID No. 1, or a sequence having at least 85%, preferably at least 90%, and more preferably at least 95% sequence identity with sequence ID No. 1, and a derivative or salt thereof, wherein said peptide has a length up to 16 amino acids, and at least one cosmetically acceptable excipient.

Advantageously, the non-therapeutic method of the present invention comprises a skin pre-treatment by Low-Frequency Sonophoresis (LFS).

## BRIEF DESCRIPTION OF FIGURES

Figure 1 shows the plot of activity of NGF1-14 at various concentrations together with the reference Negermin™ compound (Figure 1A) and the EC50 curve (Figure 1B), as described in example 1.

Figure 2 shows the scheme of treatment of the skin area interested by the experiment described in Example 2.

Figure 3 shows the levels of NGF1-14 on the epidermis (Figure 3A) and dermis (Figure 3B) measured after the treatment described in example 2.

Figure 4 shows the phosphorylation levels of TrkA receptor detected after the treatment described in example 2.

Figure 5 shows the levels of pro-Collagen I (Figure 5A) and Elastin (Figure 5B) in the dermis of rats measured after the single administration test described in example 3.

Figure 6 shows the levels of pro-Collagen I (Figure 6A) and Elastin (Figure 6B) in the dermis of rats measured after the two-weeks administration test described in example 3.

## DETAILED DESCRIPTION OF THE INVENTION

A first object of the present invention relates to a peptide comprising the sequence ID No. 1 for use in the treatment and prevention of skin aging and photo-aging. Seq. ID No. 1 according to the present invention is represented by the sequence SSSHPIFHRGEFSV.

Abbreviations of the amino acid sequences used herein are in accordance with the IUPAC-IUB nomenclature as reported in the following Table A.

Table A

| Alanine | Ala | A | Arginine | Arg | R |
|---------|-----|---|----------|-----|---|
| Asparagine | Asn | N | Aspartic acid | Asp | D |
| Cysteine | Cys | C | Glutamic acid | Glu | E |
| Glutamine | Gln | Q | Glycine | Gly | G |
| Histidine | His | H | Isoleucine | Ile | I |
| Leucine | Leu | L | Lysine | Lys | K |
| Methionine | Met | M | Phenylalanine | Phe | F |
| Proline | Pro | P | Serine | Ser | S |
| Threonine | Thr | T | Tryptophan | Trp | W |
| Tyrosine | Tyr | Y | Valine | Val | V |

Peptides according to the invention may have at least 85%, at least 90% and at least 95% sequence identity to sequence ID No. 1 when optimally aligned. Optimal alignment of the sequences may be conducted by various known methods and computerized implementation of known algorithms (e.g. BLAST, TFASTA, BESTFIT, such as in the Wisconsin Genetics Software Package, Release 7.0, Genetics Computer Group, Madison, WI). The BLAST algorithm (Altschul et al., Mol. Biol. (1990), 215, 403-410) for which software may be obtained through the National Center for Biotechnology Information www.ncbi.nlm.nih.gov/) may also be used.

"Percentage sequence identity" with respect to a peptide sequence refers to the percentage of residues that are identical in two sequences. The percent sequence identity (%SI) is calculated by the following formula:

$$\%SI = (nt-nd)x100/nt$$

wherein nt is the number of residues in the basic sequence and nd is the total number of non-identical residues in the confronted sequence when aligned so that a maximum number of amino acids are identical. Accordingly, a sequence SSSHPIFHRGDFSV will have a sequence identity of about 92.8% with the sequence ID. No 1 (nd=1 and nt=14).

The peptides according to the invention may have the same length of sequence ID. No 1 or a length up to 16 amino acids.

Variation of the amino acid sequence in the peptides comprising the sequences ID No. 1 of the present invention comprises conservative substitution of amino acids that do not influence peptide activity. The substitutions able to maintain the peptide activity are selected on the basis of (a) the efficacy in maintaining the structure of the peptide backbone in the area of substitution, such as sheet or helical three-dimensional structures, (b) the efficacy in maintaining electrical charge or hydrophobicity of the molecule in the target area, or (c) the efficacy of maintaining the bulk of the side chain.

Examples of conservative substitution belong to the group consisting of basic amino acids (arginine, lysine and histidine), acidic amino acids (glutamic acid and aspartic add), polar amino acids (glutamine and asparagine), hydrophobic amino acids (leucine, isoleucine, valine and methionine), aromatic amino acids (phenylalanine, tryptophan and tyrosine), and small amino acids (glycine, alanine, serine, and threonine).

The amino acid substitutions that do not generally alter the specific activity are known in the art of the present invention.

Most common occurred alteration are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly, and the opposite alterations. Another example of conservative substitutions are shown in the following Table C.

TABLE C

| Starting amino acid | Possible substitution | Preferred substitution |
|---------------------|----------------------|------------------------|
| Ala (A) | V; L; I | V |
| Arg (R) | K; Q; N | K |
| Asn (N) | Q; H; D, K; R | Q |

(continued)

| Starting amino acid | Possible substitution | Preferred substitution |
|---|---|---|
| Asp (D) | E; N | E |
| Cys (C) | S; A | S |
| Gln (Q) | N; E | N |
| Glu (E) | D; Q | D |
| Gly (G) | A | A |
| His (H) | N; Q; K; R | R |
| Ile (I) | L; V; M; A; F; | L |
| Leu (L) | I; V; M; A; F | I |
| Lys (K) | R; Q; N | R |
| Met (M) | L; F; I | L |
| Phe (F) | L; V; I; A; Y | Y |
| Pro (P) | A | A |
| Ser (S) | T | T |
| Thr (T) | S | S |
| Trp (W) | Y; F | Y |
| Tyr (Y) | Trp; F; T; S | F |
| Val (V) | I; L; M; F; A; | L |

Depending on its length, the peptide of the present invention may be synthesized by a method well known in the art, for example, by an automated peptide synthesizer, or produced by a genetic engineering technology. For example, a fusion gene encoding a fusion protein including a fusion partner and the peptide of the present invention is prepared by genetic engineering, and then transformed into a host cell to express the fusion protein. Thereafter, the peptide of the present invention is cleaved and isolated from the fusion protein using a protease or a compound so as to produce the desired peptide. To this end, a DNA sequence encoding amino acid residues which can be cleaved by a protease such as Factor Xa or enterokinase, or a compound such as CNBr or hydroxylamine may be inserted between the polynucleotides encoding the fusion partner and the peptide of the present invention.

The cosmetic composition of the present invention comprises a peptide comprising the sequence ID No. 1 and at least one cosmetically acceptable excipient.

The cosmetic composition of the present invention can comprise an amount of the peptide, or a derivative and/or salt thereof, ranging from 0.00000001% to 20% by weight, preferably from 0.000001 % to 15% by weight, more preferably from 0.0001% to 10% by weight, and even more preferably from 0.0001% to 5% by weight.

The cosmetic composition of the present invention can contain a variety of other optional components suitable for rendering such compositions more cosmetically or aesthetically acceptable or to provide them with addition usage benefits. Such conventional optional ingredients are well-known to those skilled in the art. These include any cosmetically acceptable ingredients such as those found in the CTFA International Cosmetic Ingredient Dictionary and Handbook, 7th edition, edited by Wenninger and McEwen, (The Cosmetic, Toiletry, and Fragrance Association, Inc., Washington, D. C., 1997). As used herein "cosmetically acceptable" means a material (e. g., compound or composition) which is suitable for use in contact with skin, hair or other suitable substrate as defined hereinbelow.

Cosmetically acceptable ingredients useful in the present invention includes cosmetically acceptable carriers, volatile and non-volatile solvents, water, and other additional ingredients, such as surfactants, preservatives, absorbents, chelating agents, lubricants, moisturizers water repellents, anti-oxidants, UV absorbers, anti-irritants, vitamins, trace metals, anti-microbial agents, perfumes, dyes and colour ingredients, and/or structuring agents.

The expression "cosmetically acceptable carrier", as used herein, means one or more compatible solid or liquid fillers, diluents, extenders and the like, which are cosmetically acceptable as defined hereinabove. The term "compatible", as used herein, means that the components of the compositions of this invention are capable of being combined with the primary actives of the present invention, and with each other, in a manner such that there is no interaction which would substantially reduce the efficacy of the composition under ordinary use situations.

The type of carrier utilized in the present invention depends on the type of product desired. The compositions useful in the present invention may be a wide variety of product forms. These include, but are not limited to, lotions, creams, gels, sticks, sprays, ointments, pastes, mousses and cosmetics (e. g., solid, semi-solid, or liquid make-up, including foundations).

These product forms may comprise several types of carriers including, but not limited to, solutions, aerosols, emulsions (including oil-in-water or water-in-oil), gels, solids, and liposomes.

The compositions of the present invention may comprise water, in different amounts depending on the form of the composition. The amount of water, if present, can range from less than 1% to more than 99% by weight with respect to the weight of total composition. The aqueous composition of the present invention are especially formulated as aqueous lotions or as water-in-oil or oil-in-water emulsions or as multiple emulsions (oil-in-water-in-oil or water-in-oil-in-water triple emulsion). Such emulsions are known and described, for example, by C. FOX in "Cosmetics and Toiletries" - November 1986 - Vol. 101 - pages 101 - 112.

Solid compositions, spray compositions, and water-in-oil creams usually comprise amounts of water lower than 10%, more preferably lower than 5% by weight with respect to the total weight of the composition. Roll-on compositions, aqueous compositions, and deodorant usually comprises amount of water of from about 15% to about 99%, more preferably from about 30% to about 90%, even more preferably about 50% to about 80%, by weight with respect to the total weight of the composition.

The compositions of the present invention may comprise one or more volatile solvent. If present, the volatile solvent or mixture of solvents will generally be at a level of from about 10% to about 90%, more preferably from about 25% to about 75%, even more preferably about 35% to about 65%, by weight with respect to the total weight of the composition. The solvents useful herein are preferably organic volatile solvents.

As used herein, "volatile" refers to substances with a significant amount of vapour pressure under ambient conditions, as is understood by those in the art.

The volatile solvents for use herein will preferably have a vapour pressure of about 2kPa or more, more preferably about 6kPa or more, at 25°C. The volatile solvents for use herein will preferably have a boiling point under normal atmosphere (1 atm) of less than about 150°C, more preferably less than about 100°C, even more preferably less than about 90°C, even more preferably still less than about 80°C.

Preferably, the volatile solvents for use herein will be relatively odourless and safe for use on human skin. Suitable volatile solvents include, but are not limited to C1-C4 alcohols, volatile silicones and mixtures thereof. Preferred volatile solvents are C1-C4 alcohols and mixtures thereof. More preferred for use herein is ethanol.

The compositions of the present invention may also comprise one or more non-volatile solvent. If present, the non-volatile solvent or mixture of solvents will generally be at a level of from about 1% to about 20%, more preferably from about 2% to about 10%, even more preferably from about 3% to about 5%, by weight with respect to the total weight of the composition. Suitable non-volatile solvents include, but are not limited to, benzyl benzoate, cetearyl alcohol, cetyl alcohol, diethyl phthalate, isopropyl myristate, dimethicone, caprylylmethicone, and mixtures thereof.

Several other additional ingredients can be present in the compositions of the present invention. These include, but are not limited to, hydrophilic polymers selected from polyethylene glycols (PEGs), polyvinylpyrrolidones (PVP), hydroxypropyl methylcellulose (HPMC) and poloxamers; UV stabilizers such as benzophenone-3; antioxidants such as tocopheryl acetate; preservatives such as phenoxyethanol, benzyl alcohol, methyl paraben, propyl paraben; pH adjusting agents such as lactic acid, citric acid, sodium citrate, succinic acid, phosphoric acid, sodium hydroxide, sodium carbonate; deodorants and anti-microbials, such as farnesol, zinc phenolsulphonate, and ethylhexylglycerin; humectants such as tribehenin, glycerine; skin conditioning agents such as allantoin; cooling agents such as trimethyl isopropyl butanamide and menthol; hair conditioning ingredients such as panthenol, panthetine, pantotheine, panthenyl ethyl ether, and combinations thereof; propellants such as propane, isopropane, butane, and isobutene; salts in general, such as potassium acetate and sodium chloride and mixtures thereof; perfumes and dyes.

If present, these additional ingredients will preferably be present at a level of less than 10%, more preferably of less than 5%, by weight with respect to the total weight of the composition.

The peptides of the present invention are able to promote the production of pro-collagen I and elastin, as well as to activate melanocytes for melanin production. Accordingly, the cosmetic composition of the present invention is especially useful for the treatment and prevention of skin aging and photo-aging.

The invention will be further described in the following examples, which do not limit the scope of the invention described in the claims.

## EXAMPLE 1

In vitro activity

NGF treatment of rat pheochromocytoma cell line (PC12) results in c-fos mRNA and protein expression (J. Milbrandt, "Nerve growth factor rapidly induces c-fos mRNA in PC12 rat pheochromocytoma cells", Proc Natl Acad Sci U S A. 1986 Jul;83(13):4789-93).

Starting from the work of Milbrandt, the Applicant monitored the activation of NFG receptor by using a clone (B9) stably transfected with the human c-fos promoter driving a luciferase reporter gene (PC12-Luci). This clone was used to set up a functional and quantitative assay for the characterization of the activity of NGF1-14. The experiments were performed in PC12-Luci cells, which endogenously express the NGF receptors, TrkA and p75. Cells were maintained in the following complete culture medium comprising:

- DMEM-F12 mixture (DMEM-F12 Glutamax; Life Technologies, GIBCO 10565-018)
- 5% fetal bovine serum (FBS, Sigma Aldrich, F7524)
- 10% horse serum (HS, Sigma Aldrich, H1138)
- 1% penicillin-streptomycin (Life Technologies, GIBCO 15140-122)

For passaging, cells were gently washed with PBS and then incubated with trypsin for 5 min at room temperature or 37°C. Media was added and cells were resuspended (6-8 times).

Experiments were performed in sestuplicate, in black 96 well previously coated with 50$\mu$g/ml collagen type I.

PC-12-Luci cells were seeded at 25000/well in 100 $\mu$l complete growth medium. 24h later, cell medium was carefully removed. Cells were then incubated with five concentrations (range 30ng/ml-300$\mu$g/ml, 1:10 serial dilutions) of NGF1-14 in standard Tyrode buffer with 0.1% BSA (50 $\mu$l/well) for 4 h at 37°C. The reference Negermin™ compound (by MimeTech Srl) has been added at 30$\mu$g/ml, as reference. The stock solution of NGF1-14 was resuspended in sterile water, aliquoted and stored at -20°C. The stock solution of Negermin™ compound was resuspended in formulation buffer and stored at -20°C.

After the incubation, the basal level of luminescence was monitored for 30 seconds, by using EnSpire multimode plate reader (PerkinElmer). Then, a Triton-Luciferin mix was added to cells (50 $\mu$l/well), in a 1:1 ratio, leading to cell lysis. The obtained luminescence signal was monitored for other 60 seconds.

The solutions and buffers mentioned above had the following composition:

- Formulation buffer: 50 mM NaH$_2$PO$_4$, 100 mM NaCl, pH 7.2,
- Tyrode buffer: 130 mM NaCl, 5 mM KCI, 2 mM CaCl$_2$, 1mM MgCl$_2$, 5 mM NaHCOs, 20 mM HEPES in water at pH 7.4; sterile filtered and stored a 4°C,
- Bovine serum albumin (BSA), fatty acid-free (Sigma, A8806); stock solution: 10% in Tyrode buffer; aliquoted and stored at -20°C,
- Triton lysis stock solution: 25 mM TRIS, 25 mM Na$_2$HPO$_4$, 2 mM DTT, 10% Glycerol, 2% TRITON X-100; aliquoted and stored at -20°C,
- Luciferin stock solution: 20 mM Tricine, 2.67 mM MgSO$_4$, 0.1 mM EDTA, 33.3 mM DTT, 270 $\mu$M Coenzyme A, 470 $\mu$M Luciferin, 530 $\mu$M ATP; aliquoted and stored at -80°C.

All luminescence values were normalized versus the basal level of luminescence. The response value was calculated as the mean of luminescence of the kinetic recorded.

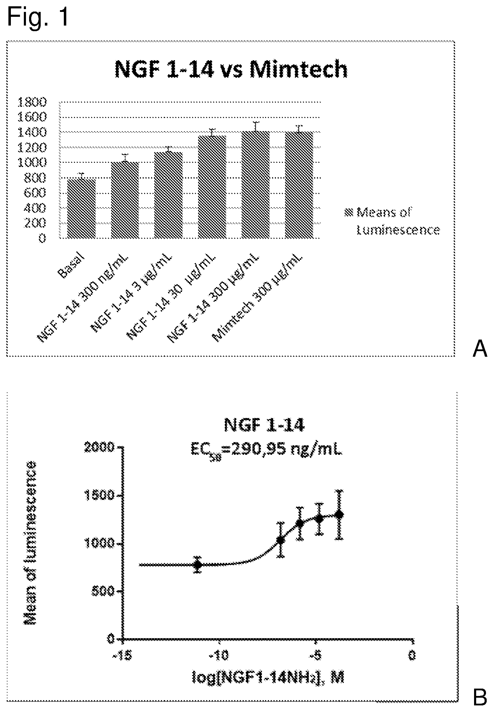

Data were further analyzed (e.g. calculation of dose-response curves and EC50 values) and graphs plotted using GraphPad Prism (GraphPad Software, Inc.). The results are illustrated in Fig. 1, showing the plot of activity of NGF1-14 at various concentrations together with the reference Negermin™ compound (Figure 1A) and the EC50 curve (Figure 1B). The results showed that NGF1-14 increases the level of basal luminescence in a dose-dependent manner (Ec50=290.95 ng/ml).

In conclusion, these results demonstrated that NGF1-14 possesses functional activity, showing an in vitro potency higher than reference standard Negermin™ compound, but lower than rhNGF (Ec50 rhNGF 1.4 ng/ml). The reduced potency is coherent with the proposed cosmetic use.

## EXAMPLE 2

Preclinical study

Distribution experiments were carried out to evaluate the ability of NGF1-14 gel preparation to penetrate the corneal stratus and reach the dermis, also by using sonophoresis-like strategies to facilitate skin penetration.

Tape-striping and ultrasounds were applied on the shaved dorsal area of female adult Sprague Dawley (SD) rats (10-12 months).

Low-Frequency Sonophoresis (LFS) was applied using a Sonics Ultrasonic Processor Model VCX 400 operating at a frequency of 20 KHz (tip displacement of about 2 cm, continuous mode) for 6 or 10 minutes, according to common clinical practices, since a prolonged use of sonophoresis is known to increase skin permeation but also to a considerable thermal damage, which is not appropriate for the use (Ref. International Journal of Health Sciences & Research 477 Vol.5; Issue: 5; May 2015: A Review on Ultrasound Parameters and Methods of Application in Transdermal Drug Delivery).

Vehicle control and NGF1-14 gel preparation were administered on the skin portion pre-treated with LFS (S) or on shaved/tape striping skin (NS).

The compositions of the vehicle control and NGF1-14 gel preparation are reported in the following Table 1.

TABLE 1

|  | Vehicle control | NGF1-14 |
|---|---|---|
| Sodium Hyaluronate based hydrogel | About 90-95% | About 90-95% |
| Polyvinyl alcohol (Sigma Aldrich Mowiol® 40-88) | 5-10% | 5-10% |
| Paraben stabilizers (Sigma Aldrich) | Less than 1% | Less than 1% |
| NGF1-14 | - | 0.1% |

Sodium Hyaluronate based hydrogel was prepared by dissolving Sodium Hyaluronate having molecular weight ranging from 50 to 150 KDa (manufactured by Contipro) in water.

Treatment was repeated after two hours, and rats were sacrificed to dissect skin samples 2 hours after the second gel/NGF mimetic application.

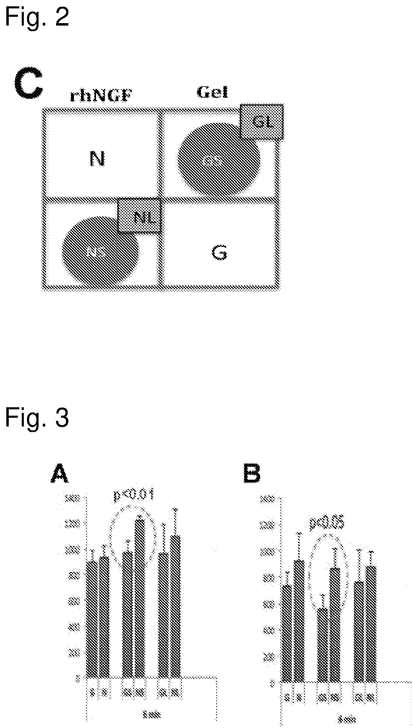

A scheme of treatment is showed in Figure 2, where N and G indicate the area treated without LFS with NGF1-14 and vehicle Gel, respectively, and NS and GS indicate the area treated with LFS with NGF1-14 and vehicle Gel, respectively. NL and GL indicate skin areas adjacent to and no overlapping LFS areas.

Epidermis and dermis from sonicated (S) and no sonicated (NS) skin areas were separated using a lancet, washed with PBS and cut in two parts. The former was stored in a sterile tube at -80°C to be processed for total protein extraction and biochemical analysis.

A skin portion was immersed in PFA 4% to be processed for subsequent histological and immunohistochemical evaluation.

The protein extract from epidermis and dermis was employed to evaluate the effect of LFS application on the ability of NGF1-14 gel preparation to penetrate the corneal stratus and reach the dermis.

Quantification of NGF 1-14 was determined by HPLC-UV, on a reverse phase C4 column, eluting with phosphate buffer (pH 7.4) and acetonitrile. The HPLC-UV method was validated for linearity, repeatability and reproducibility and recovery.

The results are showed in Figure 3A and 3B. Compared to treatment with vehicle, significant increase of NGF1-14 was found in epidermis and dermis following LFS. The efficacy of NGF mimetic/LFS application was confirmed by analyzing the expression levels and activation of the NGF survival/trophic receptor TrkA.

The effect of NGF(1-14) on the phosphorylation of TrkA was tested by Western Blot analyses of protein extracts from PC12 cell cultures treated with the peptide, by using Antibodies to P-TrkA, according to common laboratory practice.

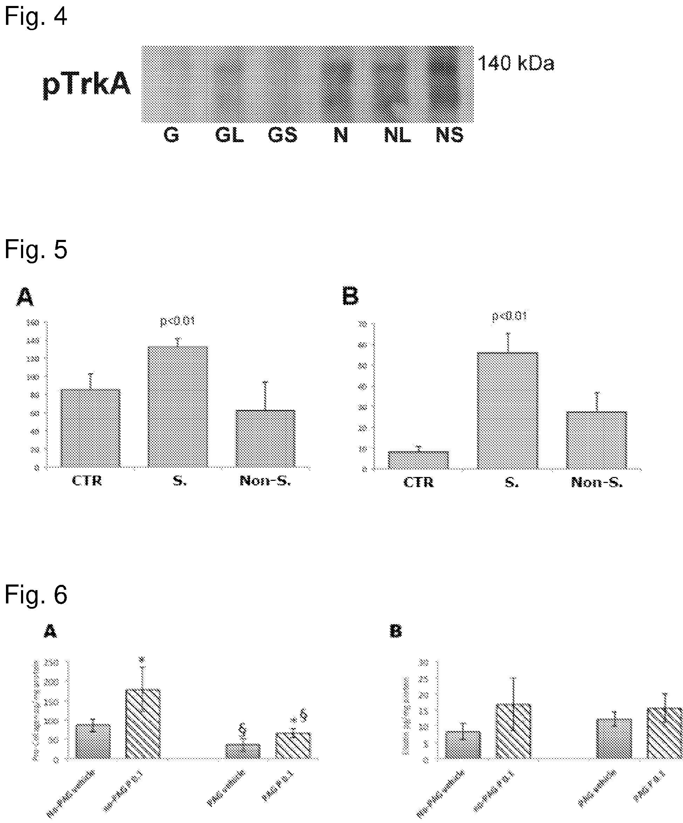

As it is showed in Figure 4 increased phosphorylation levels of TrkA were found in all dermis samples receiving NGF1-14 application, with or without LFS when compared to the related vehicle controls.

**EXAMPLE 3**

Skin aging and photo-aging

Since the proteins of extracellular matrix collagen and elastin are involved in wound repair, skin aging and photo-aging, the effect of topical application of NGF1-14 on the levels of these two proteins was also analyzed.

Pro-Collagen I and Elastin were measured by using commercial Elisa kits, first in a single administration test, followed by a two weeks administration test.

Results for the single administration test are summarized in Figure 5. A similar trend was observed by analyzing the two proteins: an increase of both pro-Collagen I (A) and Elastin (B) was detected in dermis of NGF1-14 treated rats at 0.1% dose, after sonication. The effect was less appreciable without applying the ultrasounds. A slight increase of Elastin was detected, while the value of Collagen I was comparable to the positive control value, within the experimental error.

*Single and Two weeks administration test*

The protective action of NGF1-14 at 0.1% dose (P 0.1%) was evaluated on adult skin exposed to sub-erythematous levels of UV-B (MED=60 mJ/cm$^2$ twice a day) first in a single administration test (one day) then for three weeks before the treatment. This experimental procedure is also refereed as Photo-AGing model (PAG) since it induces structural and molecular changes occurring in aging.

Topical treatment with P 0.1% or vehicle gel was applied on shaved dorsal skin of PAG and no-PAG rats for two weeks. UV-B exposure (MED=60 mJ/cm$^2$ one a day) was also performed during the last week treatment.

Rats were sacrificed and the skin was dissected using a lancet, washed with PBS and cut in two parts. The former was stored in a sterile tube at -80°C to be processed for total protein extraction and biochemical analysis. The latter was immersed in PFA 4% and processed for histological and immune-histochemical evaluation.

In agreement with the single administration study, photo-aging induced a decrease of pro-Collagen I in dermis (Figure 6A), and in detected Elastin expression (Figure 6B).

Application of P 0.1% resulted in pro-Collagen I increase in both no-PAG and PAG dermis, although the effect was more pronounced in healthy skin (Fig 6A). The changes were less significant in Elastin concentration.

## EXAMPLE 4

Tanning action

The effect of topical application of NGF1-14 on the activation of melanocytes to produce melanin has been assessed.

According to already set up protocols, primary cell cultures of normal human melanocytes were seeded in Petri dishes and cultured for a week, with and without the presence of NGF 1-14 at various concentrations. The melanin content was measured by enzyme immunoassay ELISA. An increase was observed in cultures enriched with the peptide.

## EXAMPLE 5

Skin Irritation Test

Skin Irritation Tests were performed by applying tailored assay protocols for dermal cosmetic application, namely MTT Colorimetric assay and LDH Release assay.

The MTT Colorimetric assay measures the activity of the mitochondrial enzyme succinate dehydrogenase, which is only active in living cells and reacts with MTT (colored yellow) forming a blue colored salt, whose optical density (ODn), which is proportional to the number of live cells, is quantified spectrophotometrically at a wavelength of 570 nm.

The LDH Release assay measures the amount of lactate dehydrogenase, a cytoplasmic enzyme, into the culture medium, whose release means integrity loss of the cell membrane. The amount is quantified spectrophotometrically by means of a specific reagent at a wavelength of 490 nm.

The tests were performed in triplicate with two sample (1 and 2) of NGF1-14 gel formulation at 0.3% dose, three times higher than the active one (0.1%), on EpiDerm™ tissue (MatTek Corporation, Bratislava, Slovakia) in EPI-100-ASY Assay Medium. As a positive control an 1% Triton X-100 Solution was used. As a negative control the gel matrix without peptide was used. Positive and negative controls were run in duplicate. The samples were contacted overnight for 18 hours.

The results of the tests are summarized in the following tables 2 (MTT) and 3 (LDH).

TABLE 2

|  | Average value (OD) $\pm$ SD | Normalized value $\pm$ SD |
|---|---|---|
| Negative control | 2.0 $\pm$ 0.1 | 100.0 $\pm$ 3.1 |
| Positive control | 0.7 $\pm$ 0.0 | 33.3 $\pm$ 2.0 |
| Sample 1 | 1.8 $\pm$ 0.0 | 91.6 $\pm$ 1.8 |
| Sample 2 | 1.9 $\pm$ 0.1 | 99.3 $\pm$ 3.8 |

TABLE 3

|  | Average value (OD) $\pm$ SD | Normalized value $\pm$ SD |
|---|---|---|
| Negative control | 0.13 $\pm$ 0.00 | 1.0 $\pm$ 0.0 |
| Positive control | 1.04 $\pm$ 0.04 | 8.0 $\pm$ 0.3 |
| Sample 1 | 0.24 $\pm$ 0.04 | 1.9 $\pm$ 0.3 |

(continued)

|  | Average value (OD) $\pm$ SD | Normalized value $\pm$ SD |
|---|---|---|
| Sample 2 | 0.14 $\pm$ 0.05 | 1.1 $\pm$ 0.4 |

In the MTT assay, a sample is considered "not irritant" when the vitality is higher than 70% with respect the negative control. In the LDH assay, a sample is considered "not irritant" when the value of LDH release is lower than five times the value registered for the negative control. Both samples 1 and 2 were then considered "not irritant".

## EXAMPLE 6

Skin Sensitization Test

Skin Sensitization Test were performed by applying the IL-18 Release assay with Elisa Kit.

The IL-18 Release assay measures the amount of interleukin 18 (IL-18) into the culture medium. IL-18 is a pro-inflammatory cytokine having a key-role in inducing allergic contact sensitization. The amount is quantified by direct ELISA method, i.e., the color development was directly proportional to the amount of cytokines in the culture medium.

The tests were performed in triplicate with two sample (1 and 2) of NGF1-14 gel formulation at 0.3% dose, three times higher than the active one (0.1%), on EpiDerm™ tissue (MatTek Corporation, Bratislava, Slovakia) in EPI-100-ASY Assay Medium. As a positive control a 0.15% DNCB (2,4-DiNitroChloroBenzene) solution was used. As a negative control the gel matrix without peptide was used. Positive and negative controls were run in duplicate. The samples were contacted overnight for 18 hours.

The results of the tests are summarized in the following table 4.

TABLE 4

|  | Average value (pg/ml) $\pm$ SD | Normalized value $\pm$ SD |
|---|---|---|
| Negative control | 27.0 $\pm$ 3.8 | 1.0 $\pm$ 0.1 |
| Positive control | 252.3 $\pm$ 16.4 | 9.3 $\pm$ 0.6 |
| Sample 1 | 38.3 $\pm$ 5.6 | 1.4 $\pm$ 0.2 |
| Sample 2 | 45.6 $\pm$ 8.2 | 1.7 $\pm$ 0.3 |

In the IL-18 Release assay, a sample is considered "not sensitizing" when the value of IL-18 release is lower than two times the value registered for the negative control. Both samples 1 and 2 were then considered "not sensitizing".

## EXAMPLE 7

NGF1-14 pKa and Log P and D characterization

Experimental pKa as well as partition and distribution coefficients (log P and log D) were determined using an automated potentiometric titrator Sirius T3 (Sirius Analytical Instruments Ltd., East Sussex, UK) equipped with an Ag/AgCl double junction reference pH electrode, a Sirius D-PAS spectrometer and a turbidity sensing device. The pH electrode was calibrated titrimetrically in the pH range 1.8-12.2. An overhead stirrer was used and a temperature probe monitored the temperature during the course of the titration. The titration experiments were conducted in 0.15 M KCI solution (ISA water) under an argon atmosphere at a temperature of 25 $\pm$ 1°C. All tests were performed using standardized 0.5 M KOH and 0.5 M HCI as titration reagents, for the partition coefficient tests a saturated Octanol in ISA water (5% of ISA water) was used as partition solvent. The pKa is determined by potentiometric method by pH-metric titration. The powder (around 0.5 mg) was dissolved in 1.5 ml of ISA water and the titration was performed in triplicate in the pH range 2.0 - 12.0. Log P is performed in triplicate by dissolving the powder (around 0.7 mg) in 1 ml of ISA water followed by pH metric titration in three different percentage of Octanol (generally 50%, 60%, 70%). The predicted pKa and the calculated log P for the instrumental model was performed using ACD/Percepta.

The results are illustrated in the following Table 5. The Log P value has to be considered a relative values as the peptides are never neutral molecules.

TABLE 5

| Acid pKa | 4.42 $\pm$0.05 (n=50) |
|---|---|
| Base pKa | 7.41 $\pm$0.07 (n=50) |
| Base pKa | 6.43 $\pm$0.06 (n=50) |
| Log P (w/o) | -8.50 |
| Log D (7.4) | -7.90 |

The present invention has been disclosed with particular reference to some specific embodiments thereof, but it should be understood that modifications and changes may be made by the persons skilled in the art without departing from the scope of the invention as defined in the appended claims.

**Claims**

1.    A peptide comprising the sequence ID No. 1, or a sequence having at least 85%, preferably at least 90%, and more preferably at least 95% sequence identity with sequence ID No. 1, and a derivative or salt thereof, for use in the treatment and prevention of skin aging and photo-aging, wherein said peptide has a length up to 16 amino acids.

2.    The peptide for use according to claim 1, wherein said peptide has a length up to 15 amino acids, and preferably of 14 amino acids.

3.    The peptide for use according to claim 2, wherein said peptide consists in the sequence ID No. 1, or a sequence having at least 85%, preferably at least 90%, and more preferably at least 95% sequence identity with sequence ID No. 1, and a derivative or salt thereof.

4.    The peptide for use according to any one of preceding claims 1 to 3, wherein said derivative comprises an N- and/or C-terminal of said peptide which is chemically modified or protected with an organic compound.

5.    The peptide for use according to claim 4, wherein said organic compound is selected from the group consisting of phosphoryl, glycosyl, acyl, alkyl, carboxyl, hydroxyl, biotinyl, ubiquitinyl, and amido groups.

6.    A cosmetic composition comprising a peptide as defined in any one of the preceding claims 1 to 5, and at least one cosmetically acceptable excipient for use in the treatment and prevention of skin aging and photo-aging.

7.    The cosmetic composition for use according to claim 6, wherein said composition comprise an amount of said peptide, or a derivative and/or salt thereof, ranging from 0.00000001% to 20% by weight, preferably from 0.000001 % to 15% by weight, more preferably from 0.0001% to 10% by weight, and even more preferably from 0.0001% to 5% by weight.

8.    The cosmetic composition for use according to claim 6, wherein said at least one cosmetically acceptable excipient is selected from the group consisting of cosmetically acceptable carriers, volatile and non-volatile solvents, water, surfactants, preservatives, absorbents, chelating agents, lubricants, moisturizers water repellents, anti-oxidants, UV absorbers, anti-irritants, vitamins, trace metals, anti-microbial agents, perfumes, dyes and colour ingredients, and/or structuring agents.

9.    The cosmetic composition for use according to claim 8, wherein said cosmetically acceptable carriers include solutions, aerosols, oil-in-water emulsions, water-in-oil emulsions, gels, solids, and liposomes

10.    The cosmetic composition for use according to claim 8, wherein said cosmetic composition is in the form of lotions, creams, gels, sticks, sprays, ointments, pastes, mousses and cosmetics.

11.    A non-therapeutic method for the treatment and prevention of skin aging and photo-aging in a person in need thereof, comprising the topical application of a cosmetic composition comprising a peptide as defined in any one of the preceding claims 1 to 5, and at least one cosmetically acceptable excipient.

12.    The non-therapeutic method according to claim 11, wherein said method comprises a skin pre-treatment by Low-Frequency Sonophoresis (LFS).

**Patentansprüche**

1. Peptid umfassend die Sequenz-ID Nr. 1 oder eine Sequenz mit mindestens 85%, bevorzugt mindestens 90%, und mehr bevorzugt mindestens 95% Sequenzidentität mit Sequenz-ID Nr. 1, und ein Derivat oder Salz davon, zur Verwendung bei der Behandlung und Vorbeugung von Hautalterung und Lichtalterung, wobei das Peptid eine Länge bis zu 16 Aminosäuren aufweist.

2. Peptid zur Verwendung nach Anspruch 1, wobei das Peptid eine Länge bis zu 15 Aminosäuren und bevorzugt von 14 Aminosäuren aufweist.

3. Peptid zur Verwendung nach Anspruch 2, wobei das Peptid aus der Sequenz-ID Nr. 1, oder einer Sequenz mit mindestens 85%, bevorzugt mindestens 90%, und mehr bevorzugt mindestens 95% Sequenzidentität mit Sequenz-ID Nr. 1, und einem Derivat oder Salz davon besteht.

4. Peptid zur Verwendung nach einem der vorhergehenden Ansprüche 1 bis 3, wobei das Derivat einen N- und/oder C-Terminus des Peptids umfasst, welcher mit einer organischen Verbindung chemisch modifiziert oder geschützt ist.

5. Peptid zur Verwendung nach Anspruch 4, wobei die organische Verbindung ausgewählt ist aus der Gruppe bestehend aus Phosphoryl-, Glycosyl-, Acyl-, Alkyl-, Carboxyl-, Hydroxyl-, Biotinyl-, Ubiquitinyl- und Amidogruppen.

6. Kosmetische Zusammensetzung umfassend ein Peptid wie in einem der vorhergehenden Ansprüche 1 bis 5 definiert und mindestens einen kosmetisch annehmbaren Exzipienten, zur Verwendung bei der Behandlung und Vorbeugung von Hautalterung und Lichtalterung.

7. Kosmetische Zusammensetzung zur Verwendung nach Anspruch 6, wobei die Zusammensetzung eine Menge des Peptids oder eines Derivats und/oder Salzes davon in einem Bereich von 0,00000001 Gew.-% bis 20 Gew.-%, bevorzugt von 0,000001 Gew.-% bis 15 Gew.-%, mehr bevorzugt von 0,0001 Gew.-% bis 10 Gew.-% und noch mehr bevorzugt von 0,0001 Gew.-% bis 5 Gew.-% umfasst.

8. Kosmetische Zusammensetzung zur Verwendung nach Anspruch 6, wobei der mindestens eine kosmetisch annehmbare Exzipient ausgewählt ist aus der Gruppe bestehend aus kosmetisch annehmbaren Trägern, flüchtigen und nichtflüchtigen Lösungsmitteln, Wasser, Tensiden, Konservierungsmitteln, Absorptionsmitteln, Chelatbildnern, Gleitmitteln, Feuchtigkeitsmitteln, wasserabweisenden Mitteln, Antioxidantien, UV-Absorbern, Reizhemmern, Vitaminen, Spurenmetallen, antimikrobiellen Mitteln, Parfüms, Farbstoffen und Farbbestandteilen und/oder Strukturierungsmitteln.

9. Kosmetische Zusammensetzung zur Verwendung nach Anspruch 8, wobei die kosmetisch annehmbaren Träger Lösungen, Aerosole, Öl-in-Wasser-Emulsionen, Wasser-in-Öl-Emulsionen, Gele, Feststoffe und Liposomen umfassen.

10. Kosmetische Zusammensetzung zur Verwendung nach Anspruch 8, wobei die kosmetische Zusammensetzung in der Form von Lotionen, Cremes, Gelen, Stiften, Sprays, Salben, Pasten, Mousses und Kosmetika vorliegt.

11. Nichttherapeutisches Verfahren zur Behandlung und Vorbeugung von Hautalterung und Lichtalterung bei einer Person, die dessen bedarf, umfassend die topische Anwendung einer kosmetischen Zusammensetzung umfassend ein Peptid wie in einem der vorhergehenden Ansprüche 1 bis 5 definiert und mindestens einen kosmetisch annehmbaren Exzipienten.

12. Nichttherapeutisches Verfahren nach Anspruch 11, wobei das Verfahren eine Hautvorbehandlung durch Niederfrequenz-Sonophorese (engl. Low-Frequency Sonophoresis, LFS) umfasst.

**Revendications**

1. Peptide comprenant la séquence ID. n° : 1, ou une séquence ayant au moins 85 %, préférablement au moins 90 %, et plus préférablement au moins 95 % d'identité de séquence avec SEQ. ID. n° : 1, et un dérivé ou un sel de celui-ci, pour l'utilisation dans le traitement et la prévention du vieillissement de la peau et du photo-vieillissement, dans lequel ledit peptide présente une longueur allant jusqu'à 16 acides aminés.

2. Peptide pour l'utilisation selon la revendication 1, dans lequel ledit peptide présente une longueur allant jusqu'à 15 acides aminés, et préférablement de 14 acides aminés.

3. Peptide pour l'utilisation selon la revendication 2, dans lequel ledit peptide consiste en la séquence ID. n° : 1, ou une séquence ayant au moins 85 %, préférablement au moins 90 %, et plus préférablement au moins 95 % d'identité de séquence avec SEQ. ID. n° : 1, et un dérivé ou un sel de celui-ci.

4. Peptide pour l'utilisation selon l'une quelconque des revendications précédentes de 1 à 3, dans lequel ledit dérivé comprend une extrémité N-terminale et/ou C-terminale dudit peptide qui est chimiquement modifié ou protégé par un composé organique.

5. Peptide pour l'utilisation selon la revendication 4, dans lequel ledit composé organique est sélectionné dans le groupe constitué des groupes phosphoryle, glycosyle, acyle, alkyle, carboxyle, hydroxyle, biotinyle, ubiquitinyle, et amido.

6. Composition cosmétique comprenant un peptide tel que défini dans l'une quelconque des revendications précédentes de 1 à 5, et au moins un excipient cosmétiquement acceptable pour l'utilisation dans le traitement et la prévention du vieillissement de la peau et du photo-vieillissement.

7. Composition cosmétique pour l'utilisation selon la revendication 6, dans laquelle ladite composition comprend une quantité dudit peptide, ou un dérivé et/ou un sel de celui-ci, s'étendant de 0,00000001 % à 20 % en poids, préférablement de 0,000001 % à 15 % en poids, plus préférablement de 0,0001 % à 10 % en poids, et même plus préférablement de 0,0001 % à 5 % en poids.

8. Composition cosmétique pour l'utilisation selon la revendication 6, dans laquelle ledit au moins un excipient cosmétiquement acceptable est sélectionné dans le groupe constitué des supports cosmétiquement acceptables, des solvants volatils et non volatils, de l'eau, des tensioactifs, des agents conservateurs, des absorbants, des agents chélatants, des lubrifiants, des agents hydratants, des agents hydrofuges, des agents antioxydants, des agents absorbant le rayonnement UV, des agents anti-irritants, des vitamines, des métaux sous forme de trace, des agents antimicrobiens, des parfums, des teintures et des ingrédients donnant de la couleur, et/ou des agents structurants.

9. Composition cosmétique pour l'utilisation selon la revendication 8, dans laquelle lesdits supports cosmétiquement acceptables incluent des solutions, des aérosols, des émulsions huile-dans-eau, des émulsions eau-dans-huile, des gels, des matières solides, et des liposomes.

10. Composition cosmétique pour l'utilisation selon la revendication 8, dans laquelle ladite composition cosmétique se présente sous la forme de lotions, de crèmes, de gels, de sticks, de sprays, d'onguents, de pâtes, de mousses et de produits cosmétiques.

11. Procédé non thérapeutique pour le traitement et la prévention du vieillissement de la peau et du photo-vieillissement chez une personne en ayant besoin, comprenant l'application topique d'une composition cosmétique comprenant un peptide tel que défini selon l'une quelconque des revendications précédentes de 1 à 5, et au moins un excipient cosmétiquement acceptable.

12. Procédé non thérapeutique selon la revendication 11, dans lequel ledit procédé comprend un prétraitement de la peau par sonophorèse à basse fréquence (LFS).

## Fig. 1

A

B

Fig. 2

Fig. 3

Fig. 4

pTrkA

140 kDa

G    GL    GS    N    NL    NS

Fig. 5

A

p<0.01

CTR        S.        Non-S.

B

p<0.01

CTR        S.        Non-S.

Fig. 6

A

B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004026329 A1
- WO 2011116090 A
- WO 2011049758 A1
- WO 2005019266 A2
- WO 2013065078 A2
- WO 9939728 A
- WO 2013065078 A
- KR 20170049299
- KR 20100058858
- WO 9849308 A
- CN 107286233
- CN 107973848

**Non-patent literature cited in the description**

- **PROF. RITA LEVI-MONTALCINI ; LEVI-MONTAL-CINI R.** Harvey Lect.. Zoology Institute of the Washington University of St. Louis, 1966, vol. 60, 217
- **HEFTI F.** *J. Neurobiol.*, 1994, vol. 25, 1418
- **FRICKER J.** *Lancet*, 1997, vol. 349, 480
- **OLSON L. et al.** *J. Neural Trans.: Parkinson's Disease and Dementia Section*, 1992, vol. 4, 79
- **PETTY B.G. et al.** *Annals of Neurology*, 1994, vol. 36, 244-246
- **CHAO MV ; HEMPSTEAD BL.** p75 and Trk: a two-receptor system. *Trends Neurosci.*, 1995, vol. 18, 321-6
- **MEAKIN SO ; SHOOTER EM**. The nerve growth factor family of receptors. *Trends Neurosci*, 1992, vol. 15, 323-31
- **P. DI PIETRO et al.** Immobilization of Neurotrophin Peptides on Gold Nanoparticles by Direct and Lipid-Mediated Interaction: A New Multipotential Therapeutic Nanoplatform for CNS Disorders. *ACS Omega*, 2017, vol. 2 (8), 4071-4079
- **C. SATRIANO et al.** Neurotrophin-mimicking peptides at the biointerface with gold respond to copper ion stimuli. *Physical Chemistry Chemical Physics*, 2016, vol. 18 (44), 30595-30604
- **A. TRAVAGLIA et al.** A small linear peptide encompassing the NGF N-terminus partly mimics the biological activities of the entire neurotrophin in PC12 cells. *ACS Chem Neurosci.*, 19 August 2015, vol. 6 (8), 1379-92
- **ALTSCHUL et al.** *Mol. Biol.*, 1990, vol. 215, 403-410
- CTFA International Cosmetic Ingredient Dictionary and Handbook. The Cosmetic, Toiletry, and Fragrance Association, Inc., 1997
- **C. FOX**. *Cosmetics and Toiletries*, November 1986, vol. 101, 101-112
- **J. MILBRANDT**. Nerve growth factor rapidly induces c-fos mRNA in PC12 rat pheochromocytoma cells. *Proc Natl Acad Sci U S A.*, July 1986, vol. 83 (13), 4789-93
- Ref. International Journal of Health Sciences & Research 477. *A Review on Ultrasound Parameters and Methods of Application in Transdermal Drug Delivery*, May 2015, vol. 5 (5)